# EUROPEAN PATENT APPLICATION

(11) **EP 2 174 683 A1**
(43) Date of publication of application: **14.04.2010**
(21) Application number: 08773187.3
(22) Date of filing: 15.07.2008
(51) Int. Cl.: A61M 16/16

(54) **OXYGEN HUMIDIFICATION AND DELIVERY DEVICE**

(30) Priority: 18.07.2007 CN 200710130338
(71) Applicant: Beijing Winsunny Pharmaceutical Co., Ltd., Beijing 101113 (CN)
(72) Inventor: DONG, Dongsheng, Beijing 100071 (CN)
(74) Representative: von Kreisler Selting Werner
(86) International application number: PCT/CN2008/071641
(87) International publication number: WO 2009/010006

(57) **Abstract**

An oxygen humidification and delivery device includes a case (1), humidification material (2), a seal film or seal plate (3), and an oxygen delivery tube (4). The humidification material (2) is disposed within the case (1). At least two protuberant air ports (6) are provided on the top of the case (1), one of which is air inlet, and the other is air outlet. The seal film or seal plate (3) is adapted to seal the case (1) accommodating the humidification material (2). The air ports are connected to the oxygen delivery tube (4).

## Description

### CROSS REFERENCE TO RELATED APPLICATION

This application claims the benefit of CN Patent Application No.200710130338.9, entitled "OXYGEN HUMIDIFICATION AND DELIVERY DEVICE" filed on July 18, 2007 with State Intellectual Property Office of China, the disclosures of which are hereby incorporated herein by reference.

### FIELD OF THE INVENTION

The present invention relates to an oxygen humidification and delivery device, which belongs to the technical field of medical device production.

### BACKGROUND

Conventional oxygen humidification and delivery devices include oxygen bubble humidifier. In such humidifier, oxygen is introduced below the liquid level through a vertical vent rod and produces large amounts of oxygen bubbles, which bubble through the water and then enter into a terminal delivery tube. The so-called bubble humidifier not only results in notable noise, but also generates large amount of aerosols with less than 5 micron meter in diameter. If the aerosols carry pathogens, the pathogens can be directly inhaled into respiratory tract and induce potential pulmonary infection. Furthermore, such devices are not disposable, thus disinfection and sterilization are difficult to be effectively implemented. Also, humidification liquid are mainly distilled water and are generally added in ward environment, therefore it is easily subject to contamination. These are reported in many research papers such as paper in detail in "Chinese Journal of Nosocomiology", 2002, vol. 12, No. 12.

In recent years, persons in the art have also proposed some improved oxygen humidification device, for example, in related patent applications, such as CN patent application No. 200620028624.5 entitled "Novel Wetting Bottle", CN patent application No. 200420097402.X" entitled "Soundless Oxygen Inhalation Humidification Bottle", and CN patent application No. 00203189.2 entitled "Humidifying Bottle for Oxygen Inhalation", but none of them has radically solved the above described problems.

### SUMMARY OF THE INVENTION

An Embodiment of the present invention provides an oxygen humidification and delivery device, in which no noise is produced in use and no contaminated microorganism is delivered to the respiratory tract.

The embodiment of the present invention provides an oxygen humidification and delivery device comprising a case, humidification material, a seal film or sealing sheet, and an oxygen delivery tube. The humidification material is disposed within the case. The case is provided at its top with at least two protuberant air ports, one of which is an air inlet and the other is an air outlet.

The seal film or seal plate is adapted to seal the case accommodating the humidification material. The air ports are connected with the oxygen delivery tube.

In the technical solution of the present invention, a novel structure is adopted, in which the humidification material is disposed within the case and the case is provided at its top with at least two protuberant air ports, one of which is the air inlet and the other is the air outlet; the seal film or seal sheet is adapted to seal the case accommodating the humidification material and the air ports are connected with the oxygen delivery tube. With this configuration, the humidification area for oxygen when flowing through the oxygen humidification and delivery device may be increased, the conventional bubble-type humidification is changed, which obtains an effect of noise-free humidification. The potential respiratory infections due to repeated use of the oxygen delivery device and requirements for temporary adding of the humidification liquid and the like are radically prevented; therefore the benefits of the patients who need oxygen therapy are secured.

### BRIEF DESCRIPTION OF THE DRAWINGS

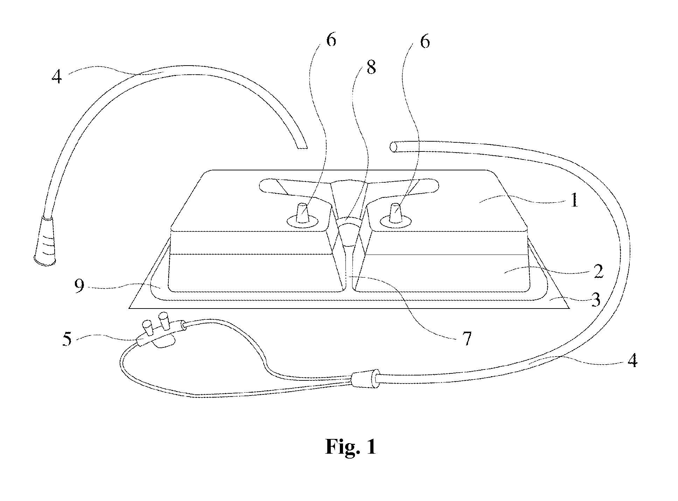

Fig. 1 is a schematic view of an oxygen humidification and delivery device provided in an embodiment of the present invention.

### Explanation of the reference numbers:

- 1:: case;
- 2:: humidification material;
- 3:: seal film;
- 4:: oxygen delivery tube;
- 5:: user terminal;
- 6:: air ports;
- 7: case bottom;
- 8:: inner peripheral portion of case bottom;
- 9:: outer peripheral portion of case bottom.

### DETAILED DESCRIPTION OF THE INVENTION

In view of the disadvantages of the conventional oxygen bubble humidifier, an embodiment of the present invention provides an oxygen humidification and delivery device including a case 1, humidification material 2, a seal film 3, and an oxygen delivery tube. Each component after sterilizing is integrated into one package so as to be disposable; the humidification material 2 is disposed within the case 1, the case 1 is provided at its top with at least two protuberant air ports 6, one of which is an air inlet and the other is an air outlet, the air ports 6 are connected with the oxygen delivery tube 4; after accommodating the humidification material 2, the case 1 is sealed by the seal film 3. During using, oxygen flows in through the air inlet, and then oxygen humidified by contacting with surface of the humidification material 2 having a large area flows out through the air outlet. The humidification material 2 may be liquid water, hydrogel or mixture of the both.

In addition to water or hydrogel, the humidification material may contain nonvolatile antiseptic agent, PH regulator, etc. to produce antiseptic effect, and may contain substances such as mint, clove, etc. to regulate smell.

Further, to improve humidification effect of the water-based humidification material, the inner surface of the case is lined with water absorbing material such as non-woven cloth or sponge, etc.

The principles, features and advantages of the present invention will be more apparent from the following illustration of application embodiments.

### Embodiment 1

The oxygen humidification and delivery device according to this embodiment is a humidification oxygen tube.

The humidification oxygen tube including:

a case, which is molded with PP (polypropylene) and has an inner volume designed according to specific circumstances, such as 300 ml; humidification material disposed within the case, which is pure water and is added with 0.05% antiseptic agent and 0.2% PH regulator, the amount of the pure water being determined according to the inner volume of the case; a seal film, which uses composite film of PP and PET (polyethylene terephthalate) having a thickness of 60 micron meter, the seal being made through heat sealing or other methods; an oxygen delivery tube, which is a PVC (Polyvinyl chloride) hose having an inner diameter of 3 mm; and user terminal, which is a nose connector, wherein the surface area of the liquid that can directly contact with oxygen when the case is placed horizontally is 150 cm², the oxygen gas flows in through an air inlet and flows out through an air outlet, the minimum distance flowed within the case is 12 cm, sterilization is made through 25KGYγ-rays irradiation.

### Embodiment 2

The oxygen humidification and delivery device according to this embodiment is a humidification oxygen mask.

The humidification oxygen mask including:

a case, which is vacuum thermoformed with PP or molded with engineering plastics and has an inner volume designed according to specific circumstances, such as 360 ml; humidification material disposed within the case, which is pure water adding with 1.2 gram seaweed gel, 0.08% antiseptic agent and 0.3% PH regulator, the amount of the pure water being determined according to the inner volume of the case; a seal film, which uses composite film of PP and PET having a thickness of 80 micron meter or engineering plastics sheet having a thickness of 1.2 mm, the seal being made through heat sealing or ultrasonic welding; an oxygen delivery tube, which is a PVC hose having an inner diameter of 5 mm; and a user terminal, which is the mask, wherein the surface area of the gel that can directly contact with oxygen when the case is placed horizontally is 180 cm², the oxygen gas flows in through an air inlet and flows out through an air outlet, the minimum distance flowed within the case is 15 cm, and sterilization is made through 25KGYγ-rays irradiation.

The above described embodiments are used to illustrate and explain the principle the present invention. It will be understood that specific embodiments of the present invention are not limited to that. To persons skilled in the art, various changes and modifications that are made without departing from the essential and the scope of the present invention are fallen within the protection scope of the present invention. Therefore the protection scope of the present invention is defined by the claims.

## Claims

1. An oxygen humidification and delivery device, **characterized by** comprising a case (1), humidification material (2), a seal film or seal plate (3), and an oxygen delivery tube (4),
wherein the humidification material (2) is disposed within the case (1), the case (1) is provided at its top with at least two protuberant air ports (6), one of which is an air inlet and the other is an air outlet; and
wherein the seal film or seal plate (3) is adapted to seal the case (1) accommodating the humidification material (2), the air ports (6) are connected with the oxygen delivery tube (4).

2. The oxygen humidification and delivery device according to claim 1, **characterized in that**, the air ports have an inner diameter of larger than 1 mm, the seal film or seal plate (3) is closely joined with an inner peripheral portion (8) and an outer peripheral portion (9) of a case bottom (7).

3. The oxygen humidification and delivery device according to claim 2, **characterized in that**, the case (1) has an inner volume of larger than 100 ml.

4. The oxygen humidification and delivery device according to claim 2, **characterized in that**, the distance within the case (1) between the air inlet and the air outlet is larger than or equal to 6 cm.

5. The oxygen humidification and delivery device according to claim 1, **characterized in that**, the seal film or seal plate (3) has a thickness of larger than or equal to 30 micron meter.

6. The oxygen humidification and delivery device according to claim 1, **characterized in that**, the humidification material (2) is liquid water, hydrogel or mixture of the both.

7. The oxygen humidification and delivery device according to claim 6, **characterized in that**, a surface area of the humidification material contacting directly with oxygen is larger than 65 cm², a distance from the liquid surface to the air ports at the top of the case is larger than or equal to 2 mm.

8. The oxygen humidification and delivery device according to claim 6, **characterized in that**, if the humidification material is the liquid water, the liquid water is added with antiseptic agent and PH regulator.

9. The oxygen humidification and delivery device according to claim 1 or 6, **characterized in that**, an inner surface of the case is lined with water absorbing material including non-woven cloth and sponge.

10. The oxygen humidification and delivery device according to claim 6, **characterized in that**, if the humidification material is the hydrogel, water content of the hydrogel is lager than or equal to 75% in weight.
